# EUROPÄISCHE PATENTSCHRIFT

(11) **EP 1 064 028 B1**
(45) Veröffentlichungstag und Bekanntmachung des Hinweises auf die Patenterteilung: **19.12.2001**
(21) Anmeldenummer: 99952074.5
(22) Anmeldetag: 12.03.1999
(51) Int. Cl.: A61L 2/06

(54) **DESINFEKTION VON EBENEN UND RAUMBILDENDEN FLÄCHEN UNTER NUTZUNG VON WASSERDAMPF**
DISINFECTING FLAT SURFACES WHICH FORM ROOMS, USING WATER VAPOUR
DESINFECTION DE SURFACES PLANES ET FORMANT DES LOCAUX AU MOYEN DE VAPEUR D'EAU

(30) Priorität: 17.03.1998 DE 19811587
(43) Veröffentlichungstag der Anmeldung: 03.01.2001
(73) Patentinhaber: Schäfer, Martin, 80637 München (DE); Beurer, Genoveva, 80997 München (DE)
(72) Erfinder: Keim, Bettina, 80335 München (DE); Schäfer, Martin, 80637 München (DE)
(86) Internationale Anmeldenummer: DE9900691
(87) Internationale Veröffentlichungsnummer: WO9959644

(56) Entgegenhaltungen:
- EP-A- 0 827 709
- DE-A- 4 108 538

## Beschreibung

Um die Übertragung krankmachender und unerwünschter Mikroorganismen auf Mensch und Tier zu vermeiden, wird in vielfältiger Weise eine Desinfektion von Oberflächen vorgenommen. Am verbreitetsten ist die Desinfektion mit Chemikalien, deren Einsatz zusammen mit der Vergabe von Antibiotika in der Vergangenheit neben den bekannten umweltbelastenden Nachteilen zunehmend zu einer Resistenzentwicklung von Mikroorganismen geführt hat .

So ist besonders in Krankenhäusern die weitere Entwicklung multiresistenter Krankheitserreger gefürchtet, die nicht mehr auf verschiedene oder kombiniert angewendete Desinfektionsmittel in der gewünschten Weise reagieren.

Weiter als Methode anerkannt und besonders für kleine Gegenstände wie z.B. medizinische Instrumente ist die thermische Desinfektion verbreitet, für die jedoch so lange Einwirkzeiten und hohe Temperaturen gefordert sind, daß ihre Anwendung in vielen Bereichen, insbesondere bei raumbildenden Flächen unwirtschaftlich oder unausführbar ist.

Es wäre jedoch in vielen Fällen wünschenswert, daß in hygienerelevanten Bereichen, wie Krankenhäusern, Großküchen, der Tierhaltung, der Lebensmittelherstellung und deren Bevorratung, sowie für Sanitärräume usw. ein möglichst einfach und ohne Schadstoffe arbeitendes Desinfektionsgerät einsetzbar ist, das alle krankmachenden und unerwünschten Mikroorganismen ausreichend beseitigt.

Hierfür gibt es Hinweise daß eine technisch einfache und kostengünstige Dampfreinigung als neue mögliche Desinfektionsmethode in Frage kommen kann.

Die bisher hierzu offenbarten Anwendungsbeispiele gehen davon aus, daß die Temperatur und der Druck eines Dampfreinigers eine desinfizierende Wirkung hervorrufen, sofern die Dampfeinwirkzeit ausreichend bemessen ist.

Die in der Vergangenheit von verschiedenen Herstellern immer wieder getätigten Werbeaussagen einer angeblichen Desinfektionswirkung mußten jedoch meist zurückgenommen werden, weil zufällig erzielte Desinfektionsergebnisse einer wissenschaftlichen Nachprüfung nicht standhielten und somit das Erfordernis einer für ein Desinfektionsverfahren unerläßlichen Zuverlässigkeit nicht gegeben war.

Eine 1994 initiierte Doktorarbeit am Institut für Tierhygiene, Verhaltenskunde und Tierschutz der Ludwig-Maximilians-Universität München untersuchte die Dampfreinigung als mögliche Desinfektionsmethode.

Die hierzu auf dem 6. Hohenheimer Seminar vom 23.- 24.09.1996 in der Tagung " Umwelt- und Tierhygiene " von A. Haas, S. Platz und J. Unshelm veröffentlichten Untersuchungsergebnisse führten zur Schlußfolgerung,
daß die bislang für ein thermisches Desinfektionsverfahren als notwendig angesehene Einwirkzeit von Wasserdampf deutlich nach unten in den Bereich 5-10 Sekunden reduziert werden kann. Vorausgesetzt hierfür ist jedoch neben dem getesteten Gerät (Uninova Hausgeräte GmbH) mit bestimmten technischen Merkmalen ein vor der Dampfaustrittsöffnung vorgespanntes Tuch und weiter ein Mindestabstand von ca. 2,5 cm von dem Tuch zum Objektträger.

Die hier nur unter Laborbedingungen erzielten Untersuchungsergebnisse ließen jedoch u.a. die Frage nach einer praxisgerechten, d.h. auch wirtschaftlich durchführbaren Anwendung insbesondere für größere Flächen offen.

Aus der Offenlegungsschrift DE 4108538 A 1 ist ein Verfahren zur thermischen Desinfektion von Oberflächen, insbesondere abgeschlossenen durchströmbaren Räumen bekannt, bei der ein aufgeheiztes, nebelförmiges und strömungsfähiges Medium erzeugt und mit zu desinfizierenden Oberflächen in Kontakt gebracht wird. Hierdurch wird offenbart, daß eine mit dem Erreger Pseudomonas aeruginosa kontaminierte Rohrleitung nach einer Beaufschlagung mit Wasserdampf als nebelförmiges Medium mit einer Temperatur zwischen 70° und 90° über einen Zeitraum von nur wenigen Minuten bakterienfrei war.

Dieses Verfahren erstreckt sich vorwiegend auf Spülvorgänge von Rohrleitungen mit Wasserdampf, nicht jedoch auf die obengenannte nur durch reines Wasser vorgegebene thermische Desinfektion größerer Flächen, da in der genannten Offenbarungsschrift vorgeschlagen wird, dem Wasser auch Desinfektionsmittel beizugeben, bzw. mit Desinfektionsmitteln zu arbeiten.

Eine von B. Bullemer am 15.11.1993 zur Klärung des naturwissenschaftlichen Hintergrundes veröffentlichte Darstellung zur Wirkungsweise des Dampfreinigers kommt zu dem Ergebnis, daß die energieliefernde Reaktion der Dampfreinigung die Rekombination der Dampfmoleküle zu Wasser ist. Aus gleicher Quelle wird am 10.04.1995 eine hypothermische Energiezufuhr durch kondensierenden Wasserdampf als alternative Desinfektionsmethode formuliert.

Als großes Problem stellte sich jedoch die Tatsache heraus, daß sich eine ausreichende Kenntnis der Hauptsätze der Thermodynamik beim Benutzer nicht vermitteln läßt. Selbst die obengenannte Dr. Arbeit zur Klärung der desinfizierenden Wirkung der Dampfreiniger geht auf das physikalische Wirkungsprinzip nicht näher ein und zeigt in der genannten Veröffentlichung Untersuchungsergebnisse unter Laborbedingungen, womit ständig sich ändernde Außenmilieubedingungen, wie durch beispielsweise unterschiedliche Temperatur- und Druckverhältnisse bei zudem unterschiedlichen Oberflächen verursacht, unberücksichtigt bleiben.

Der Erfindung liegt daher die Aufgabe zugrunde, ein Verfahren und eine Vorrichtung zur Durchführung des Verfahrens der eingangs genannten Art derart zu gestatten, daß zunächst eine wirtschaftlich durchführbare Desinfektion ebener und raumbildender Flächen bei möglichst kurzer Einwirkzeit des genutzten überhitzten Wasserdampfes ermöglicht wird und daß weiterhin die Zuverlässigkeit bei sich ständig ändernden Anwendungsbedingungen wesentlich verbessert und dabei das Wirkungsspektrum für möglichst viele Mikroorganismen erweitert wird.

Zur Lösung dieser Aufgabe ist erfindungsgemäß vorgesehen, den Wasserdampf über einen speziell ausgebildeten Desinfektionskopf zu führen.

Der Desinfektionskopf soll beweglich sein, über ein Halteteil mit Handgriff zunächst den wasserdampfemittierenden Schlauch aufnehmen, dann nach einer Vorkammer einen Düsenstock aufnehmen, der einerseits einen als optimal ermittelten Abstand von ca. 2,5 cm zur Desinfektionsfläche hält, andererseits mit dem vorderen Gehäuseteil des Desinfektionskopfes und der Objektfläche eine Dampfkammer bildet.

Der Desinfektionskopf wird von einer Absaugglocke umfaßt und saugt das entstandene Dampfkondensat über am Desinfektionskopf angeordnete Absaugdüsen ab.

Die Absaugdüsen münden in einen gemeinsamen Absaugschlauch im Inneren des den Desinfektionskopf tragenden Kunststoff-Halteteils und führen das Kondensgut in einen Auffangbehälter, von dort aus wird es in die Ausgangsverdampferzelle des Gerätes wieder eingebracht und sterilisiert.

Ein wesentliches Element des Desinfektionskopfes ist dabei der Düsenstock, durch den der Wasserdampf derart geführt wird, daß er als wassertröpfchenfreier Dampf die zu desinfizierende Fläche erreicht.

Der Düsenstock regelt den Hauptdampfstrom dadurch, daß er ihn zunächst abbremst und ihn auf eine auch wirtschaftlich sinnvoll zu nutzende Fläche verteilt.

Gleichzeitig verhindert der Düsenstock ein zu heftiges Auftreffen des Dampfes auf die zu desinfizierende Fläche und damit ein unerwünschtes Verwirbeln der Mikroorganismen. Weiterhin wird durch das Aufspalten des Dampfstromes in viele Dampfkanäle die Anzahl der Kondensationsvorgänge in der Damptkammer erhöht.

Da bei jedem Kondensationsvorgang ein mehrfaches der mittleren therinischen Energie des Siedepunktes des Wassers auf der von Mikroorganismen besiedelten Desinfektionsoberfläche abgeladen wird, bedeutet eine erhöhte Anzahl von Durchbrüchen durch den Düsenstock eine Verbesserung der für den Desinfektionserfolg maßgeblichen Energieausschöpfung.

Es stellte sich heraus, daß mit zunehmender Anzahl der Durchbrüche die Einwirkzeit verkürzt werden konnte. Überprüft wurden die Ergebnisse mit Testkulturen von Staphylococcus aureus und Pseudomonas aeruginosa und zwar unter folgenden unterschiedlichen Außenmilieubedingungen und verschiedenen Oberflächen: Holzoberflächen in Kühl-räumen mit 6° C. Temperatur, Metalloberflächen in Operationsräumen mit 17° C. Temperatur und künstlich erzeugtem Druck und durch den Einsatz von Chemikalien aufgerauhte Kunststoffoberflächen von Luftschächten eines für die Lebensmittelherstellung genutzten Reinraumes bei 23°C.

In Weiterausbildung der Erfindung wird ein Düsenstock vorgeschlagen der im Verbund mit dem genannten Desinfektionskopf ausreichend desinfiziert und darüber hinaus die dazu erforderliche Einwirkzeit des Dampfes auf unter 3 Sekunden reduziert.

Hierbei ist der Düsenstock ca. 2,5 cm stark und wird aus 4 Teilplatten gebildet, die zu einem Träger zusammengefaßt sind. Die vier Teilplatten werden durch ca. 1 mm breite Spalten getrennt. die durch die waagerechten Durchbrüche durchströmenden Wasserdampfströme aufgefüllt werden. Hierbei wird nochmals ein Teil der kinetischen Energie des durchströmenden Wasserdampfes umgewandelt und steht nach Austrittsöffnung in die vordere Dampfkammer in anderer Energieform für die eigentliche Aufgabe der Desinfektion zur Verfügung.

Die waagerechten Durchbrüche sind vollflächig, aber in gleichmäßigen Abständen von ca. 7 mm verteilt und sind bei einer Durchbruchsöffnung von ca. 0,5 mm im Durchmesser mit einer aufgedampften Metalloberfläche versehen.

Insgesamt wurde eine Durchbruchstläche von ca. 4 Promille zu einer Düscnstocksfläche als sehr effektive Dampföffnungsfläche ermittelt. Nachdem die desinfizierende Wirkung von Silber im Wasser seit langem bekannt war und im Vergleich zu anderen getesteten Materialien auch die beste Oberflächenresistenz gegenüber dem durchströmenden Wasserdampf zeigte, wurde Silber für die Beschichtung der

Durchbrüche verwendet.

In einer weileren Ausgestaltung des Erfindungsgedankens wird vorgeschlagen, den Düsenstock als ganzes, oder deren Teilplatten, die aus Keramik oder hitzebeständigem Kunststoff bestehen, zu beheizen. Die dafür erforderliche Energie kann über eine im Halteteil verlaufende Leitung zugetührt werden.

In Weiterbildung der Erfindung wird für die Ausgestaltung des Düsenstocks vorgeschlagen, die Außenflächen, soweit sie vom Wasserdampf berührt sind und die senkrechten Innenflächen der die Teilplatten trennenden Spalten mit einem weiteren Edelmetall, vorzugsweise Gold oder Platin zu beschichten. Die Vorteile dieser Beschichtung bestehen zunächst darin, daß beide Edelmetalle sich für die Verarbeitung im Verbund mit Keramik oder Kunststoff und silberbedampften Durchbrüchen als gut geeignet erweisen, korrosionsbeständig sind, und nach längerer Benutzung weder sichtbare Abnutzungen oder Verformungen zeigen.

Obwohl Wasserdampf im Regelfall rückstandsfrei abtrocknet, zeigte sich bei Versuchsreihen, daß bei unbeschichteten Keramik- oder Kunststoffoberflächen nach etwa zwei Stunden Betriebszeit die Durchbrüche sich verstopften, ebenso die Innen- und Außenflächen des Düsenstocks einen zunehmenden Belag zeigten, der die ansonsten exakte Ausbildung der laminaren Strömung behinderte.

Ähnlich unerwünschte Ergebnisse bildeten sich bei Verwendung anderer Metalle, so daß Gold und Platin auch längerfristig die günstigsten Reaktionsergebnisse zeigen. In einer weiteren Ausgestaltung der Erfindung wird statt des bisher rechteckig ausgeführten Desinfektionskopfes mit entsprechend angepaßtem Düsenstock nunmehr ein ovaler Desinfektionskopf vorgeschlagen. der in einer runden Öffnung einen zylindrisch ausgebildeten Düsenstock mit den Maßen von ca. 2.5 x 7 cm aufweist. Eine zweite Öffnung nimmt im Desinfektionskopf den Absaugkanal auf. Diese Ausgestaltung ist nach dem oben beschriebenen Wirkungsprinzip unter Nutzung von kondensierendem Wasserdampf für die Desinfektion schwer zugänglicher Stellen wie z.B. Fugen und Falze vorgesehen.

Der ovale Desinfektionskopf mündet nach Umfassung des runden Düsenstocks in eine sich verjüngende, ca. 2,5 cm lange Düse.

Als weitere Ausgestaltung kann diese ovale Düse gerade, gebogen mit verschiedenen Neigungswinkeln, oder auch um mehr als 250° gekrümmt ausgebildet sein. Line derartige Ausführung ist geeignet, eine Desinfektion durch Löcher erreichbarer abgehängter Deckenteile zu ermöglichen.

Die Öffnung für den Absaugkanal beginnt ca. 0.7 cm vor Düsenende.

In einer weiteren Ausgestaltung der Erfindung wird vorgeschlagen, das entstandene Dampfkondensat abzusaugen und in die Ausgangsverdampferzelle zurückzuführen.

In einer vorteilhaften Ausgestaltung wird der beschriebene rechteckige Desinfektionskopf von einer vorzugsweise aus durchsichtigem Kunststoff hergestellten Absaugglocke umfaßt. Die Absaugglocke ist in ihrer Grundausführung der äußeren Form des Desinfektionskopfes angepaßt und weist im Auflagebereich zu der zu desinfizierenden Fläche rotierende Gleitkugeln oder Rollen auf, die die Arbeit des Benutzers erleichtern, sowie femer die Oberflächen und den Desinfektionskopf schützen. Der Abstand zwischen Düsenstock und Oberfläche wird durch die Gleitkugeln nur unwesentlich verändert,

Jeweilige Absaugdüsen mit einem seitlichen Öffnungswinkel von ca. 135° zum Desinfektionskopf befinden sich an den Schmalseiten und/- oder unterhalb des Desinfektionskopfes und münden in Absaugkanäle, die sich vorzugsweise im Inneren des Haltestücks vereinen und schließlich in einem gemeinsamen Absauggang in einen Auffangbehälter führen.

In zweckmäßiger Ausgestaltung der Erfindung ist vorgesehen, daß die Absaugfunktion über einen am Kunststoff-Halteteil angeordneten und mit dem Daumen vom Benutzer bedienbaren Schiebe-/ und /- oder Kippschalter gesteuert wird, wobei eine Funktion dem Absaugen entspricht, die andere dem Bedampfen.

Zusätzlich ist ein optisches Signal auf dem Desinfektionskopf sinnvoll.

In besonders vorteilhafter Ausgestaltung der Erfindung ist vorgesehen, daß durch den Absaugvorgang auch evtl. überlebende Mikroorganismen mit abgesaugt und beseitigt werden. Es ist zweckmäßig, das Dampfkondensat über einen Grob- und Feinfilter in einen Auffangbehälter zu saugen, der vom Volumen kleiner sein sollte als das Volumen der Ausgangsverdampferzelle. Der Auffangbehälter und die Ausgangsverdampferzelle sind in einem Gerät zusammengefaßt, wobei der Auffangbehälter leicht herausnehmbar ist und durch das System selbst desinfiziert werden kann.

Es ist weiterhin vorgesehen, daß das im Auffangbehälter angesammelte Kondensgut über eine zweite Absaugpumpe mittels einer Zuführung in die (Ausgangs)/-verdampferzelle eingebracht wird, in der das Kondensgut mit den darin gelösten und evtl. überlebenden Mikroorganismen beim anschließenden Wiederaufheizen auf den notwendigen Arbeitsdruck automatisch sterilisiert wird.

Nach Beendigung des Wiederaufheizvorganges wird der Wasserdampf erneut dem Desinfektionskopf zugeführt.

Ein besonderer Vorteil dieser Vorrichtung liegt darin, daß auch thermoresistentere Mikroorganismen, wie z.B. Sporenbildner mit erfaßt werden, ohne die wirtschaftlich so bedeutsame kurze Einwirkzeit des Wasserdampfes nach Durchführung des ersten oben beschriebenen Verfahrensschrittes wieder aufheben zu müssen. Der Sterilisationsschritt erweitert somit das Wirkungsspektrum der Vorrichtung und somit den Anwendungsbereich.

Ein weiterer Vorzug besteht darin, daß beim Zerstören der Zellmembranen freiwerdende toxische Stoffe unschädlich gemacht werden und nicht zuletzt eine Verbesserung des Gesundtheitsschutzes für die Benutzer.

Von Vorteil ist ferner, daß die Ausgangsverdampferzelle eine vom Benutzer erreichbare Kesselablaßschraube aufweist und periodisch innen gereinigt werden kann. Ein besonderer Vorzug der Erfindung liegt in der indizierten Wassererspamis und der längeren Arbeitsfähigkeit des in einem Gerät kombinierten Desinfektions-/ und

Sterilisationssystems. Ergänzende Chemikalien sind zudem überflüssig.

Ein weiterer Vorteil besteht in der größeren Akzeptanz der Benutzer in der Krankenhaus- und Tierhygiene, wo die Sterilisation seit langem in der Praxis verankert ist, und sich das Verfahren nahtlos in bestehende Desinfektionsverfahren eingliedern ließe. Weiterhin wird auch im Lebensmittelbereich das Problem von Parasilenbefall und deren Entsorgung (z.B. auch der Eier) hygienisch unbedenklicher gelöst. Insgesamt erfüllt das vorgeschlagene Verfahren in vorteilhafter Weise alle Anforderungen, die an ein Desinfektionsverfahren zu stellen sind.

Es ist zuverlässig, wirtschaftlich, materialverträglich, benutzerfreundlich und hat ein breites, rasch und irreversibles wirkendes Spektrum. Außerdem ist es unschädlich für Mensch und Natur.

Es ist auch möglich, daß nach dem erfindungsgemäßen Verfahren eine Desinfektion und anschließende Sterilisation von hygienisch besonders bedeutsamen Oberflächen und Gegenständen erfolgt, wie z.B. Beatmungssystemen, Herz-Kreislauf-Maschinen, Kathedern, ganz allgemein Medizingeräte, Operationsschleusen, Entbindungsstationen, Intensivstationen und Infektionsstationen.

Bedarfsweise ist es zweckmäßig den Desinfektionskopf gegenüber der bisherigen Darstellung zu vergrößern, um bedarfsweise größere Düsenstöcke einsetzen zu können, um auch größere Flächen in kurzer Zeit zu desinfizieren.

Weitere Vorteile und Einzelheiten der Erfindung ergeben sich aus der nachfolgenden Beschreibung einiger Ausführungsbeispiele sowie anhand der schematisierten Zeichnungen.

Es zeigen :
- Fig. 1: einen Querschnitt eines Desinfektionskopfes mit dem eingesetzten Düsenstock, einem Halteteil, einem zuführenden Dampfschlauch, einer Absaugglocke, einer Desinfektionsoberfläche sowie einem Absaugschlauch
- Fig. 2: eine Aufsicht eines Desinfektionskopfes mit einem eingesetzten Düsenstock, seitlich aufgesetzte Absaugdüsen, sowie angedeutet die Ausbildung einer laminaren Strömung
- Fig. 3: eine Ansicht eines Düsenstocks in rechteckiger Ausführung
- Fig. 4: a. eine Ansicht eines Desinfektionskopfes in ovaler Form
b. eine Ansicht eines runden Desinfektionskopfes
c. eine Ansicht gekrümmt ausgebildeter Düsenformen
- Fig. 5: einen Querschnitt durch einen rechteckigen Düsenstock
- Fig. 6: eine Ansicht eines rechteckigen Desinfektionskopfes von unten
- Fig. 7: eine schematisierte Darstellung des kombinierten Desinfektions- und Sterilisationsverfahrens

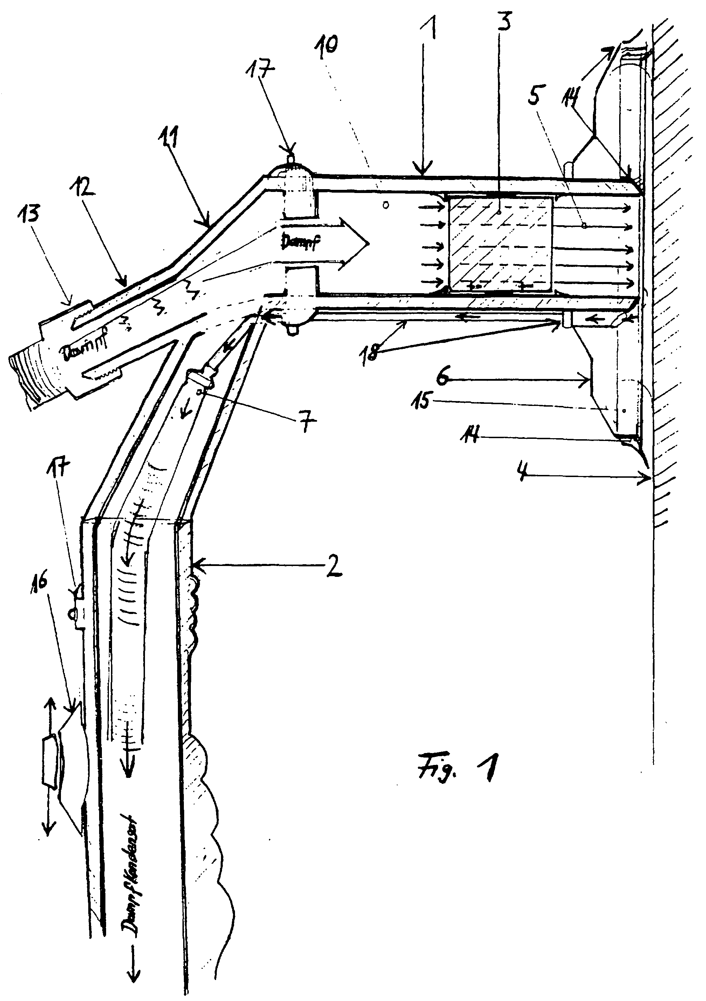

In Fig. 1 ist eine spezielle Ausführungsform eines Desinfektionskopfesl dargestellt, der von einem Kunststoff- Halteteil 2 getragen wird und im Zentrum nach einer Vorkammer 10 einen eingesetzen Düsenstock 3 enthält, durch den der aus einem Dampfeinlaßstutzen 12 emittierende Wasserdampf derart geführt ist, daß er als möglichst wassertröpfchenfreier Dampf bei laminarer Strömung auf die dem Düsenstock gegenüberliegende zu desinfizierende Fläche 4 auftrifft.

Der Düsenstock 3 bildet zusammen mit dem vorderen Gehäuseteil des Desinfektionskopfes 1 und der zu desinfizierenden Fläche 4 eine im wesentlichen geschlossene Dampfkammer 5, in der die Verdampfungsenergie ihre desinfizierende Wirkung entfaltet. Die Absaugglocke 6 nimmt das Dampfkondensat und die darin gelösten Mikroorganismen auf, und führt das Dampfkondensat über seitlich oder unterhalb des Desinfektionskopfes 1 angeordnete Absaugkanäle 18 in das Innnere des Kunststoff-Halteteils 2. Im Auflagebereich der Absaugglocke 6 laufen rotierende Gleitkugeln 15 in einer Federung, um auch Oberflächenunebenheiten ausgleichen zu können und halten dabei den Desinfektionskopf 1 auf Abstand zu der zu desinfizierenden Fläche 4. Die Absaugglocke 6 ist zusätzlich mit einem Bürstenkranz oder einem beweglichen Kunststoffband 14 versehen. Der am Kunststoff-Halteteil 2 angeordnete Schalter 16 kann durch Schiebe- oder Kippbewegung nach vorne das Bedampfen auslösen. Hierbei zeigt eine Lampe 17 die Dampfeinwirkzeit an.

Die entsprechende Bewegung nach hinten veranlaßt das Dampfabsaugen.

Bedampfen und Absaugen sind in getrennten Schlauchsystemen geführt, um den Absaugmechanismus zu erleichtern. Dem Kunststoff-Halteteil 2 können auch verschieden ausgestaltete Desinfektionsköpfe aufgesetzt werden, die über ein Kugelgelenk beweglich sind.

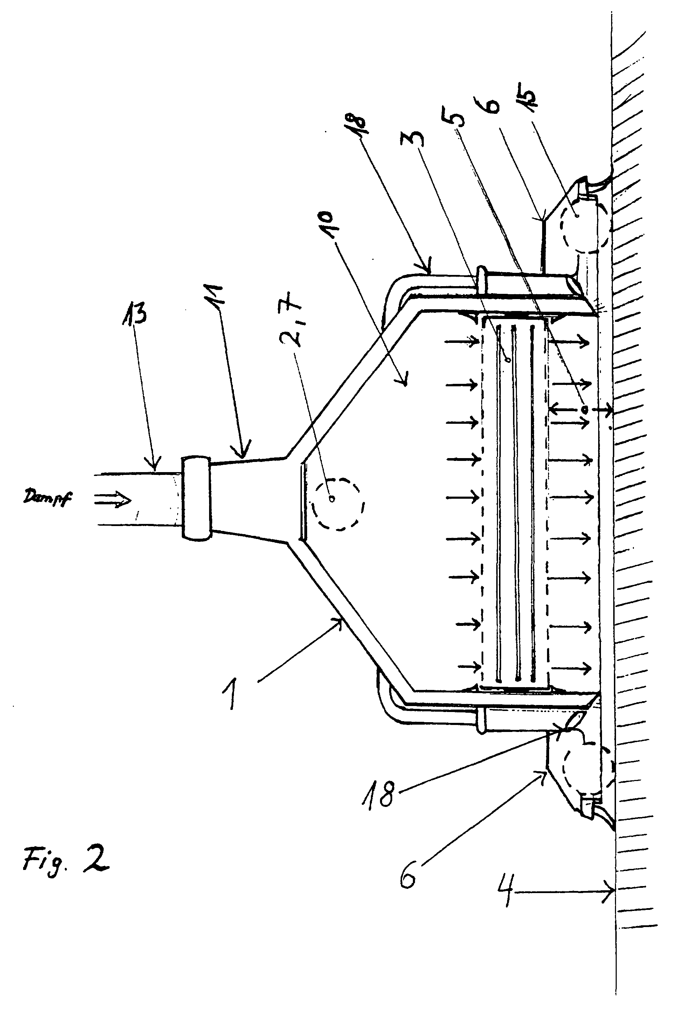

Fig. 2 zeigt in einer Draufsicht die Dampfkammer 5 mit einem Abstand von ca 2,5 cm zwischen der zu desinfizierenden Fläche 4 und dem gegenüberliegenden Düsenstock 3. Weiterhin sind an den Seiten des Desinfektionskopfes 1 die Absaugglocke 6 mit den darin laufenden Gleitkugeln 15 schematisch dargestellt.

Die seitlich angeordneten Absaugkanäle 18 münden unterhalb des Desinfektionskopfes in das Innere des Kunststoff-Halteteils 2.

Weiter dargestellt ist die Aufteilung des Düsenstocks 3 in vier Teilplatten mit einem Abstand von ca. 1 mm, um den Wirkungsgrad des Wasserdampfes für die Desinfektion zu verbessern.

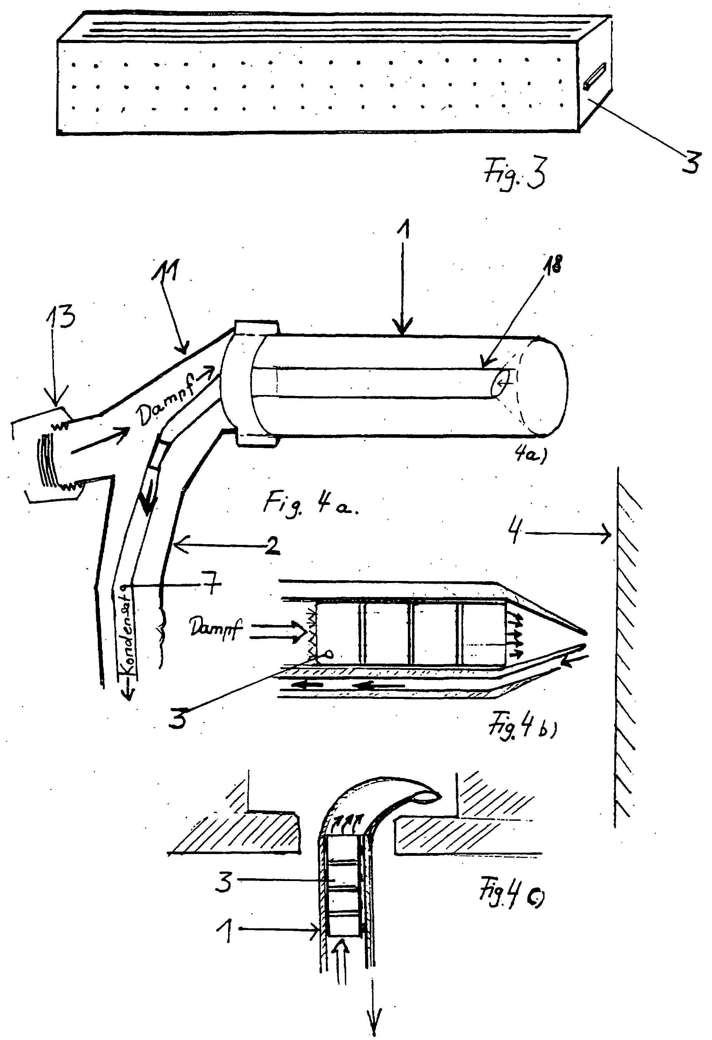

Fig.3 zeigt die perspektivische Frontansicht eines Düsenstocks 3, um die gleichmäßige Verteilung der Durchbrüche zu verdeutlichen, sowie eine seitliche Führung, die den Kontakt zu einer nicht näher dargestellten Heizleitung herstellt und der Verankerung des Düsenstocks 3 im Desinfektionskopf 1 dient.

Fig. 4a zeigt einen oval ausgestalteten Desinfektionskopf 1 mit seitlich verlaufenden Absaugkanälen 18, der mit einer entsprechend verlängerten Ausführung des Wasserdampfeinlaßstutzens 11 zur Desinfektion von Schläuchen oder Rohren dient.

Fig. 4b zeigt einen Desinfektionskopf 1, der sich für das Desinfizieren von Armaturen, Lüftungsgittern und Apparaten eignet. Durch das unverzügliche Absaugen des Kondensats aus dem durch den Einsatz des beschriebenen Düsenstocks 3 ohnehin sehr trockenen Dampf verbleibt keine Feuchtigkeit am Ort des Geschehens.

Fig. 4c zeigt die Ausgestaltung des Desinfektionskopfes 1 für die Lösung eines besonderen Problems in Infektionsstationen. In vielen Krankenhäusern sind abgehängte Lochdecken, die außer mit einer relativ giftigen Gasvernebelung für ein anderes Desinfektionsverfahren praktisch nicht zugänglich sind, dort eingesetzt, wo erhöhte Anforderungen für die Abwesenheit krankmachender Mikroorganismen bestehen. Der hier gezeigte Desinfektionskopf 1 wird außerdem in Kabel- und Leitungs-Schächten, die über Kopf verlaufen, in besonders wichtigen Bereichen z.B. der Intensivmedizin eingesetzt. Es stellte sich heraus. daß aus solchen Schächten dort bereits vorhandenes Kondensgut zusätzlich abgesaugt werden kann.

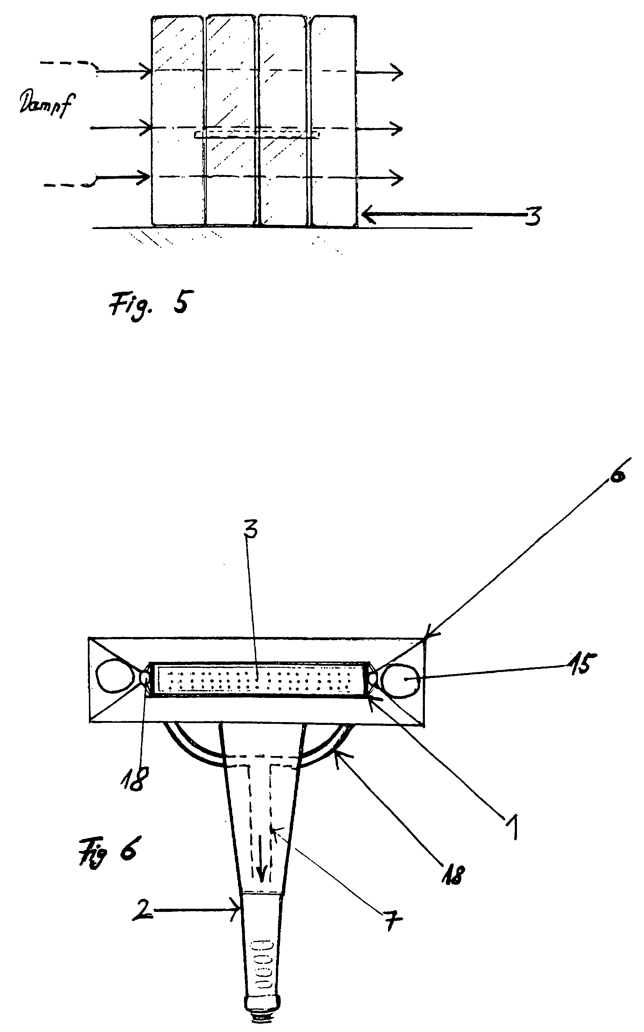

Fig. 5 zeigt den Querschnitt durch einen Düsenstock 3 mit einer Aufspaltung in vier gleichstarke Scheiben, sowie die waagerechte Laufrichtung der silberbedampften Durchbrüche.

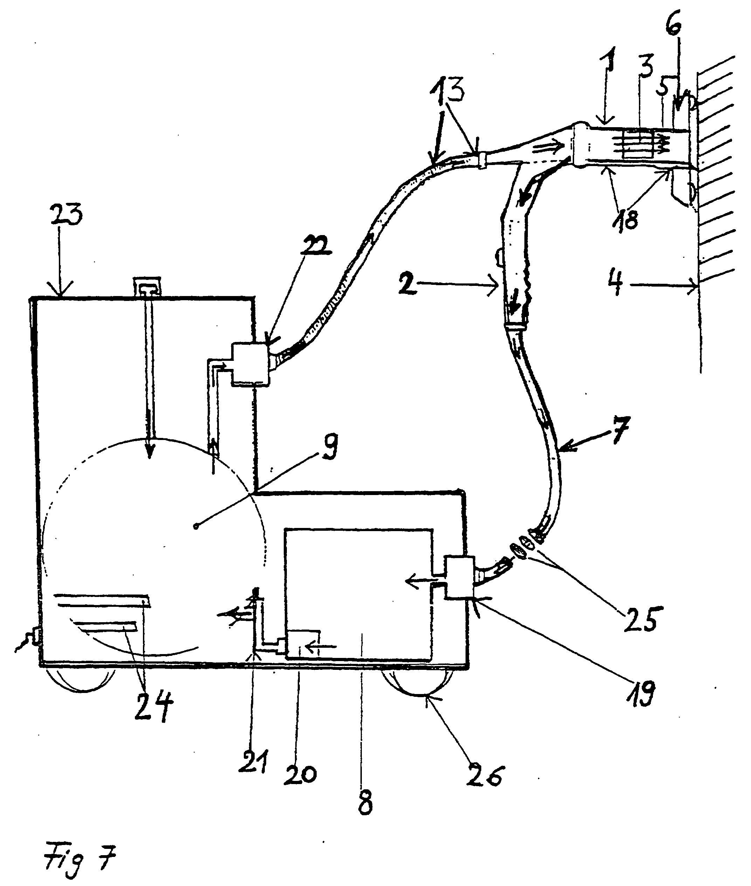

Fig. 6 zeigt den Desinfektionskopf 1 von unten. Die mit seitlich angeordneten Gleitkugeln 15 aufliegende Absaugglocke 6 zeigt Ausformungen des vorzugsweise durchsichtigen Kunststoffs, die die Absaugung des Kondensats in die Absaugkanäle 18 erleichtern. Das Kondensat wird von dort weiter in den Absaugschlauch 7 geführt.

Fig. 7 zeigt den vollständigen Bedampfungs- und Absaugungskreislauf, wobei das Bedampfen dem Desinfizieren und das Absaugen dem anschließenden Sterilisieren zugeordnet ist.

Der von der Ausganesverdampferzelle 9 emittierte Wasserdampf wird über den Desinfektionskopf 1 durch einen Düsenstock 3 geführt, verändert in der Dampfkammer 5 seinen Aggregatzustand und verrichtet bei der Abgabe seiner in der Ausgangsverdampferzelle 9 aufgenommenen Energie feinstverteilte und örtlich und zeitlich hochkonzentrierte Desinfektionsarbeit und wird mit Hilfe zweier Absaugpumpen 19 und 20 nach zweckmäßiger Grob- und Feinfilterung 25 über einen Auffang-behälter 8 und von dort aus über eine Zuführung 21 wieder in seinen Ursprungsort, in die Ausgangsverdampferzelle 9 zurückgeführt.

Die im Dampfkondensat mitgeführten Mikroorganismen, deren Bestandteile oder Reaktionsprodukte werden beim anschließenden Aufheizvorgang in der Ausgangsverdampferzelle 9 sterilisiert.

Die einzelnen Bauteile, so die Ausgangsverdampferzelle 9 mit den Heizelementen 24 und einem Dampfauslaßventil 22, sowie der über eine Zuleitung 21 mit der Ausgangsvcrdampferzelle 9 verbundene Auffangbehälter 8 mit Absaugpumpen 19 und 20 sind zu einem auf Räder 26 gelagerten mobilen Gerät 23 zusammengefaßt. Die hygienischen Vorteile des geschlossenen Kreislaufbetriebes werden noch unterstützt von einer hohen Benutzerfreundlichkeit und einer die Ressourcen Wasser und Energie sparsam verbrauchenden Technik.

### Übersicht der Bezifferung der Zeichnungen

- 1.: Desinfektionskopf
- 2.: Kunststoff-Halteteil
- 3.: Düsenstock
- 4.: zu desinfizierende Fläche
- 5.: Dampfkammer
- 6.: Absaugglocke
- 7.: Absaugschlauch
- 8.: Auffangbehälter
- 9.: Ausgangsverdampferzelle
- 10.: Vorkammer
- 11.: Einlaßstutzen
- 12.: Zuführung
- 13.: Zuführschlauch
- 14.: Kunststoffband oder Bürstenkranz
- 15.: Gleitkugeln
- 16.: Funktionsschalter Bedampfen-Absaugen
- 17.: Signallampe
- 18.: Absaugdüsen, -kanal
- 19.: Absaugpumpe
- 20.: Absaugpumpe
- 21.: Zuführung
- 22.: Dampfauslaßventil
- 23.: mobiles Gerät
- 24.: Heizelemente
- 25.: Grob- und Feinfilter
- 26.: Räder

## Patentansprüche

1. Verfahren zur Desinfektion von ebenen und raumbildenden Flächen unter Nutzung von Wasserdampf der mit der zu desinfizierenden Fläche in Kontakt gebracht wird, wobei der von einer Ausgangsverdampferzelle (9) erzeugte Wasserdampf über einen in einem Desinfektionskopf (1) befindlichen Düsenstock (3) geführt wird und bei einem vorgegebenen Abstand als wassertröpfchenfreier Dampf bei laminarer Strömung von dem Düsenstock (3) direkt auf die zu desinfizierende Fläche (4) auftrifft, wobei der Desinfektionskopf (1) mit der zu desinfizierenden Fläche eine Dampfkammer (5) bildet und nach erfolgter Kondensation das Dampfkondensat über eine dem Desinfektionskopf (1) aufgesetzte Absaugglocke (6) abgesaugt und über einen Auffangbehälter(8) in die Ausgangsverdampferzelle (9) eingebracht und durch Aufheizen sterilisiert und über die Ausgangsverdampferzelle (9) erneut dem Desinfektionskopf (1) zugeführt wird.

2. Mobile Vorrichtung zur Durchführung des Verfahrens nach Anspruch 1 umfassend, einen Düsenstock (3), der in einen Desinfektionskopf eingesetzt ist, über den Desinfektionskopf ist die Absaugglocke (6) mit den Absaugkanälen (18) verbunden aufgesetzt und der Desinfektionskopf (1) ist beweglich an einem Halteteil (2) befestigt, dem Halteteil ist ein Zuführschlauch (13) für den Wasserdampf und ein mit den Absaugkanälen (18) verbundener Absaugschlauch (7) zugeordnet, der Zuführschlauch (13) ist über ein Dampfauslaßventil (22) mit der Heizelemente (24) aufweisenden Ausgangsverdampferzelle (9) und der Absaugschlauch (7) über eine Absaugpumpe (19) mit dem Auffangbehälter (8) und weiterhin der Auffangbehälter über eine weitere Absaugpumpe (20) und eine Zuführung (21) mit der Ausgangs-Verdampferzelle verbunden, wobei der Düsenstock so ausgebildet ist und der Wasserdampf durch ihn so geführt wird, daß er als wassertröpfchenfreier Dampf beï laminarer Strömung direkt auf die zu desinfizierende Fläche auftrifft.

3. Vorrichtung nach dem Anspruch 2 **dadurch gekennzeichnet, daß** der Düsenstock (3) aus mehreren, vorzugsweise vier Teilscheiben besteht, die zu einem Träger zusammengefaßt sind, und der eine Stärke von ca 2,5 cm aufweist und über die ganze Fläche Durchbrüche von runder Form mit einem Durchmesser von etwa 0,5 mm aufweist und aus Keramik oder Kunststoff gefertigt ist.

4. Vorrichtung nach Anspruch 3 **dadurch gekennzeichnet, daß** der Düsenstock (3) bei unterschiedlicher Größe rechteckige und zylindrische Grundformen aufweist und beheizbar ist.

5. Vorrichtung nach einem der Ansprüche 2-4 **dadurch gekennzeichnet, daß** die Außenflächen und die senkrechten Innenflächen des Düsenstocks (3) mit Edelmetall, vorzugsweise Gold oder Platin beschichtet sind.

6. Vorrichtung nach einem der Ansprüche 3-5 **dadurch gekennzeichnet, daß** die Durchbrüche des Düsenstocks (3) eine aufgedampfte Edelmetalloberfläche aufweisen, vorzugsweise durch Aufdampfen von Silber.

7. Vorrichtung nach Anspruch 2 **dadurch gekennzeichnet, daß** der Desinfektionskopf (1) aus einem Kunststoffgehäuse besteht, daß eine Öffnung den Düsenstock (3) aufnimmt und gegenüberliegend der Öffnung ein Wasserdampfeinlaßstutzen (11) angeordnet ist, auf dem das Kunststoff-Halteteil (2) mit einer Dampfzuführung (12) und einem Schlauchanschluß (13) aufgesetzt ist.

8. Vorrichtung nach Anspruch 7 **dadurch gekennzeichnet, daß** der Wasserdampf-einlaßstutzen (11) und somit das Kunststoff-Halteteil (2) mit dem Desinfektionskopf (1) beweglich verbunden ist, vorzugsweise über ein Kugelgelenk.

9. Vorrichtung nach einem der bisherigen **dadurch gekennzeichnet, daß** der Desinfektionskopf (1) im Gehäusebereich der Öffnung zu der zu desinfizierenden Fläche (4) ein Bürsten- oder Kunststoffband (14) aufweist.

10. Vorrichtung nach einem der bisherigen **dadurch gekennzeichnet, daß** die Absaugglocke (6) im Auflagebereich zur desinfizierenden Fläche (4) Gleitkugeln (15) oder Rollen aufweist.

11. Vorrichtung nach einem der bisherigen Vorrichtungsansprüche **dadurch gekennzeichnet, daß** eine vom Benutzer regelbare Zeitautomatik die Absaugfunktion durch einen am Kunststoff-Halteteil (2) angeordeneten Schalter (16) steuert, wobei eine Lampe (17) die Dampfeinwirkzeit anzeigt.

12. Vorrichtung nach einem der bisherigen Vorrichtungsansprüche **dadurch gekennzeichnet, daß** am Desinfektionskopf (1) Absaugkanäle (18) angeordnet sind, deren Öffnungen einen geeigneten Abstand von der zu desinfizierenden Fläche (4) aufweisen.

13. Vorrichtung nach einem der bisherigen Vorrichtungsansprüche **dadurch gekennzeichnet, daß** die Absaugkanäle (18) aus einzelnen Düsen bestehen, die in Absaugschläuche einmünden und sich im Inneren des Kunststoff-Halteteils (2) zu einem gemeinsamen Absaugschlauch (7) verbinden.

14. Vorrichtung nach einem der bisherigen Vorrichtungsansprüche **dadurch gekennzeichnet, daß** das Kondensat über Grob- und Feinfilter (25) geführt und über eine Absaugpumpe (19) in einen Auffangbehälter (8) abgesaugt wird.

15. Vorrichtung nach einem der bisherigen Vorrichtungsansprüche **dadurch gekennzeichnet, daß** der Auffangbehälter (8) ein im Verhältnis zur Ausgangsverdampferzelle (9) reduziertes Volumen hat, wobei ein Signal zusätzlich das Erreichen einer bestimmten Füllmenge aus Kondensgut anzeigt.

## Claims

1. Process for the disinfection of flat and enclosing surfaces using steam which comes into contact with the surface to be disinfected, **characterised in that** the steam generated by an initial evaporator cell (9) is fed through a nozzle assembly (3) in a disinfection head (1) and meets the surface to be disinfected (4), which is at a specific distance away, as steam which is free of any drops of water and with a laminar flow, whereby the disinfection head (1) forms a steam chamber (5) with the surface to be disinfected (4) and **in that**, once the steam has condensed, the condensation is sucked away via a suction bell (6) placed over the disinfection head (1) and fed first into a collection container (8), then into the initial evaporator cell (9), where it is sterilised by heating and fed from the initial evaporator cell (9) back to the disinfection head (1).

2. Mobile device to carry out the procedure according to claim 1 that comprises a nozzle assembly (3) set into a disinfection head (1) and has a suction bell (6) connected to suction tubes (18) placed over the disinfection head and in which the disinfection head is attached to a holder (2) such that it can move, while an inlet hose (13) for steam and an outlet hose (7), which is connected to the suction tubes (18), are attached to the holder, with the inlet hose (13) being connected over a steam release valve (22) to the initial evaporator cell (9), which has heating elements (24), and with the inlet hose (7) being connected to the collection container (8) over a suction pump (19) while the collection container in turn is connected over another suction pump (20) and an inlet (21) to the initial evaporator cell, and whereby the nozzle assembly is shaped in such a manner that the steam is conducted through it so that it meets the surface to be disinfected as steam which is free of any drops of water and with laminar flow.

3. Device in accordance with claim 2 **characterised in that** the nozzle assembly (3) consists of several plates, preferably four, which join together to form a carrier, has a thickness of approx. 2.5 cm, has round openings with a diameter of around 0.5 mm across the whole of its surface and is made of plastic or ceramic material.

4. Device in accordance with claim 3, **characterised in that** the nozzle assembly (3) can be rectangular or cylindrical and different sizes, and can be heated.

5. Device in accordance with one of the claims 2 - 4, **characterised in that** the external surfaces and the vertical internal surfaces of the nozzle assembly (3) arc coated with a precious metal, preferably gold or platinum.

6. Device in accordance with one of claims 3 - 5, **characterised in that** the openings on the nozzle assembly (3) exhibit a vacuum coated precious metal surface, preferably vacuum coated in silver.

7. Device in accordance with claim 2, **characterised in that** the disinfection head (1) is made of a plastic casing which has an opening to accommodate the nozzle assembly (3) and that opposite the opening there is a steam inlet spout (11) on which the plastic holder (2), with its steam inlet (12) and its hose connection (13), rests.

8. Device in accordance with claim 7, **characterised in that** the steam inlet spout (11) and therefore the plastic holder (2) are attached to the disinfection head (1) such that they can move, preferably by means of a ball joint.

9. Device in accordance with one of the above device claims, **characterised in that** the disinfection head (1) exhibits a brush or plastic band (14) in the area of the casing around the opening to the surface to be disinfected (4).

10. Device in accordance with one of the above device claims, **characterised in that** the suction bell (6) has sliding bearings (15) or rollers at the point where it comes into contact with the surface to be disinfected (4).

11. Device in accordance with one of the above device claims, **characterised in that** an automatic time function operated by the user controls the suction function by means of a switch (16) on the plastic holder (2), whereby a lamp (17) displays the effective time for the steam.

12. Device in accordance with one of the above device claims, **characterised in that**, on the disinfection head (1), there are suction nozzles (18), the opening angle of which is more than 100 degrees and which are around 2 cm from the surface to be disinfected (4).

13. Device in accordance with one of the above device claims, **characterised in that** the suction nozzles (18) join up to suction hoses and meet inside the plastic holder section (2) to form a single suction hose (7).

14. Device in accordance with one of the above device claims, **characterised in that** the condensed material is passed through coarse and fine filters (25) and sucked by a suction pump (19) into a collection container (8).

15. Device in accordance with one of the above device claims, **characterised in that** the collection container (8) has a lesser volume than that of the initial evaporator cell (9), whereby a signal indicates when the condensed material has reached a certain level.

## Revendications

1. Procédé de désinfection des surfaces planes et creuses utilisant la vapeur d'eau mise en contact avec la surface à désinfecter, où la vapeur d'eau produite par la cellule de vaporisation de sortie (9) est dirigée par l'intermédiaire d'un porte-gicleur (3) placé dans l'embout de désinfection (1), directement sur la surface à désinfecter et, pour un intervalle donné, il se dégage un écoulement laminaire de vapeur exempt de toute goutte d'eau ; ici l'embout de désinfection (1) constitue avec la surface à désinfecter une chambre à vapeur (5) dont les condensats sont aspirés par une buse d'aspiration (6) placée sur l'embout de désinfection (1) et sont renvoyés vers la cellule de vaporisation de sortie (9) par l'intermédiaire d'un réservoir collecteur (8) où la stérilisation est réalisée par chauffage ; la vapeur régénérée est conduite à nouveau vers l'embout de désinfection (1) à partir de la cellule de vaporisation de sortie (9).

2. Dispositif mobile pour l'exécution du procédé selon la revendication 1, comprend un porte-gicleur (3), placé dans l'embout de désinfection, la buse d'aspiration (6) raccordée aux conduits d'aspiration (18), et qui se trouve au dessus de l'embout de désinfection, lequel embout est fixé de façon articulée à un support (2) auquel est adjoint un tuyau d'alimentation (13) pour la vapeur d'eau et un tuyau d'aspiration (7) raccordé aux conduits d'aspiration (18) ; le tuyau d'alimentation (13) est raccordé par l'intermédiaire d'une soupape d'échappement (22) à la cellule de vaporisation de sortie (9) qui présente des éléments chauffants (24) et le tuyau d'aspiration (7) qui est, quant à lui, raccordé par l'intermédiaire d'une pompe aspirante (19) au réservoir collecteur (8), lequel réservoir est lui-même raccordé par une pompe aspirante supplémentaire (20) et un conduit (21) à la cellule de vaporisation de sortie ; ici le porte-gicleur est constitué de telle sorte et la vapeur envoyée par son intermédiaire de telle façon, que l'écoulement laminaire de vapeur, exempt de toute goutte d'eau, parvient directement sur la surface à désinfecter.

3. Dispositif selon la revendication 2 **caractérisé par le fait que** le porte-gicleur (3) est composé de plusieurs, de préférence quatre plateaux diviseurs, lesquels sont groupés de façon à former un support et qui a une épaisseur d'environ 2,5 cm et présente des ouvertures circulaires sur toute sa surface, lesquelles ouvertures ont un diamètre d'environ 0,5 mm et sont réalisées en céramique ou en matière plastique.

4. Dispositif selon la revendication 3 **caractérisé par le fait que** le porte-gicleur (3) présente des formes rectangulaires et cylindriques dans le cadre de tailles différentes et qu'il peut être chauffé.

5. Dispositif selon l'une des revendications 2-4 **caractérisé par le fait que** les surfaces extérieures et surfaces verticales intérieures du porte-gicleur (3) sont recouvertes avec du métal précieux, de préférence avec de l'or ou du platine.

6. Dispositif selon l'une des revendications 3-5 **caractérisé par le fait que** les ouvertures du porte-gicleur (3) ont une surface métallisée au métal précieux, de préférence par une couche d'argent appliquée.

7. Dispositif selon la revendication 2 **caractérisé par le fait que** l'embout de désinfection (1) est composé d'un boîtier en matière plastique, que le porte-gicleur (3) est logé dans une ouverture et qu'une buse d'admission de vapeur d'eau (11) est située en face de cette ouverture, sur laquelle est fixé le support en matière plastique (2) avec un conduit à vapeur (12) et un raccord de tuyau (13).

8. Dispositif selon la revendication 7 **caractérisé par le fait que** la buse d'admission de vapeur d'eau (11) et par conséquent le support en plastique (2), sont raccordés de façon articulée avec l'embout de désinfection (1), de préférence par une articulation sphérique.

9. Dispositif selon l'une des anciennes revendications de dispositif **caractérisé par le fait que** l'embout de désinfection (1) est équipé d'une brosse ou d'un ruban en plastique (14) au niveau du boîtier de l'ouverture pour la surface à désinfecter (4).

10. Dispositif selon l'une des anciennes revendications de dispositif **caractérisé par le fait que** la buse d'aspiration (6) est équipée de billes (15) ou de roulements au niveau de la zone d'appui de la surface à désinfecter (4).

11. Dispositif selon l'une des anciennes revendications de dispositif **caractérisé par le fait qu'**un système automatique à choix préalable d'ouverture commandé par l'utilisateur régule la fonction d'aspiration au moyen d'un commutateur placé sur le support en plastique (2) et où une diode (17) indique la durée d'action de la vapeur.

12. Dispositif selon l'une des anciennes revendications de dispositif **caractérisé par le fait que** des conduits d'aspiration (18) sont raccordés à l'embout de désinfection (1) et dont les ouvertures présentent un écart adapté par rapport à la surface à désinfecter (4).

13. Dispositif selon l'une des anciennes revendications de dispositif **caractérisé par le fait que** les conduits d'aspiration (18) sont composés de buses individuelles qui débouchent au niveau des tuyaux d'aspiration et se regroupent en un tuyau d'aspiration commun (7) à l'intérieur du support en plastique (2).

14. Dispositif selon l'une des anciennes revendications de dispositif **caractérisé par le fait que** le condensat est conduit à travers un préfiltre et un filtre fin (25) et aspiré par une pompe aspirante (19) vers un réservoir collecteur (8).

15. Dispositif selon l'une des anciennes revendications de dispositif **caractérisé par le fait que** le réservoir collecteur (8) présente un volume réduit par rapport à la cellule de vaporisation de sortie (9), où en plus un signal indique l'obtention d'un volume déterminé de remplissage de vapeur.
